# EUROPEAN PATENT SPECIFICATION

(11) **EP 2 962 689 B1**
(45) Date of publication and mention of the grant of the patent: **11.04.2018**
(21) Application number: 15173625.3
(22) Date of filing: 24.06.2015
(51) Int. Cl.: A61K 31/137, A61K 31/4174, A61K 31/498, A61P 17/00, A61K 9/06, A61K 9/00

(54) **METHOD OF TREATING FLUSHING ASSOCIATED WITH CARCINOID TUMORS AND CARCINOID SYNDROME**
VERFAHREN ZUR BEHANDLUNG VON SPÜLUNGEN IM ZUSAMMENHANG MIT KARZINOIDEN TUMOREN UND KARZINOIDSYNDROM
PROCEDE DE TRAITEMENT DES BOUFFEES DE CHALEUR ASSOCIEES A DES TUMEURS CARCINOÏDES ET AU SYNDROME CARCINOÏDE

(30) Priority: 30.06.2014 US 201462019067 P
(43) Date of publication of application: 06.01.2016
(73) Proprietor: Galderma S.A., 1000 Lausanne 30 Grey (CH)
(72) Inventor: Brown, Philip Manton, Dallas, TX Texas 75205 (US)
(74) Representative: V.O.

(56) References cited:
- WO-A1-2011/053487
- US-A1- 2006 264 515
- US-A1- 2012 101 141

## Description

### CROSS-REFERENCE TO RELATED APPLICATION

This application claims priority from U.S. Provisional Application Serial No. 62/019,067, filed June 30, 2014.

### BACKGROUND OF THE INVENTION

Carcinoid tumors are rare cancerous tumors usually affecting the gastrointestinal tract or lungs. The tumors sometimes lead to the secretion of chemicals into the bloodstream causing a variety of symptoms collectively called carcinoid syndrome. About 5% to about 10% of patients with carcinoid tumors exhibit the symptoms of carcinoid syndrome. The most common symptom of carcinoid syndrome is flushing of the face, neck, and/or upper chest. Flushing includes change in color, usually redness, of the affected areas and sometimes sensations of heat.

There is a need for new therapies to treat flushing associated with carcinoid tumors and carcinoid syndrome in order to improve the quality of life of patients with carcinoid tumors and carcinoid syndrome.

US 2012/0101141 A1 discloses topical gel compositions comprising an alpha adrenergic receptor agonist selected from the group consisting of oxymetazoline, phenylephrine, methoxamine, tetrahydrozoline, naphazoline, xylometazoline, epinephrine and norepinephrine for the treatment of skin disorders. Mention is made of the following skin disorders: rosacea, telangiectasia, psoriasis, purpura, erythema of acne, eczema, non-rosacea-related inflammation of the skin, flushing, skin sagging, creasing and/or wrinkling. US 2006/0264515 A1 discloses topical formulations comprising an alpha adrenergic receptor agonist for the treatment of telangiectasias. WO 2011/053487 A1 discloses a method of treating or preventing acute erythema by topical administration of an effective amount of an alpha adrenergic receptor agonist or pharmaceutically acceptable salt thereof.

### SUMMARY OF THE INVENTION

The invention relates to a pharmaceutical composition for use in the treatment of flushing associated with carcinoid tumors and carcinoid syndrome in a patient in need thereof, which composition comprises an alpha adrenergic receptor agonist, pharmaceutically acceptable salt thereof, or combinations thereof; and a pharmaceutically acceptable carrier, wherein the composition is to be topically applied to the site of the flushing. In one embodiment, the site of the flushing is the face or the neck.

Preferably, the alpha adrenergic receptor agonist is an alpha-1 adrenergic receptor agonist or an alpha-2 adrenergic receptor agonist. The preferred alpha-1 adrenergic receptor agonist or alpha-2 adrenergic receptor agonist is brimonidine, tetrahydrozoline, naphazoline, xylometazoline, epinephrine, norepinephrine, oxymetazoline, phenylephrine, or methoxamine. The most preferred alpha-2 adrenergic receptor agonist is brimonidine or a pharmaceutically acceptable salt thereof. Brimonidine or a pharmaceutically acceptable salt thereof is preferably present in an amount of from about 0.5% by weight to about 5% by weight of the composition, more preferably from about 0.5% by weight to about 2% by weight of the composition. In a preferred embodiment, the brimonidine or pharmaceutically acceptable salt thereof is present in an amount of about 0.33% by weight of the composition.

Preferably, the pharmaceutically acceptable carrier is a gel, cream, ointment, lotion, or emulsion. Gels are preferred carriers.

### DETAILED DESCRIPTION

The invention relates to of the aforementioned pharmaceutical composition for use in the treatment of flushing associated with carcinoid tumors and carcinoid syndrome in a patient in need thereof. Carcinoid syndrome is a pattern of symptoms seen in about 5% to about 10% of patients with carcinoid tumors. The most prevalent symptom of carcinoid syndrome is flushing of the face, neck, and/or upper chest. Flushing involves the skin on the face, neck, and/or upper chest changing colors ranging from pink to red to purple and, sometimes, sensations of heat. Redness of the skin is most commonly associated with flushing. Flushing episodes may last a few minutes to a few hours or longer.

A pharmaceutical composition for use in the treatment of flushing associated with carcinoid tumors and carcinoid syndrome according to the present invention includes an effective amount of one or more alpha adrenergic receptor agonist or pharmaceutically acceptable salt thereof, and a pharmaceutically acceptable carrier, wherein the composition is to be applied topically to the skin at the site of the flushing.

Alpha adrenergic receptor agonists are well known in the art. In a preferred embodiment, the alpha adrenergic receptor agonist may be an alpha-1 or alpha-2 adrenergic receptor agonist. The alpha adrenergic receptor agonists included in the composition for use according to the present invention may or may not show selectivity for either the alpha-1 or alpha-2 adrenergic receptors. For example, some may be considered as being both alpha-1 and alpha-2 adrenergic receptor agonists. More preferably, the alpha adrenergic receptor agonist may be a selective alpha-1 or a selective alpha-2 adrenergic receptor agonist.

Examples of selective alpha-1 adrenergic receptor agonists include oxymetazoline, phenylephrine, and methoxamine. Examples of selective alpha-2 adrenergic receptor agonists include brimonidine, tetrahydrozoline, naphazoline, xylometazoline, epinephrine, and norepinephrine.

The chemical structures of some selective alpha-1 and selective alpha-2 adrenergic receptor agonists are shown below.

| Chemical Structure | Name |
|---|---|
| 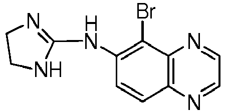 | (5-Bromo-quinoxalin-6-yl)-(4,5-dihydro-1H-imidazol-2-yl)-amine (Brimonidine) |
| 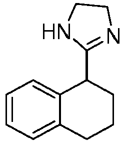 | Tetrahydrozoline |
| 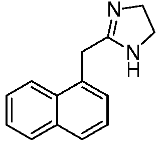 | Naphazoline |
| 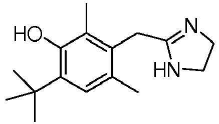 | Oxymetazoline |
| 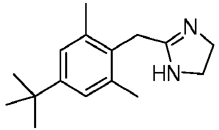 | Xylometazoline |
| 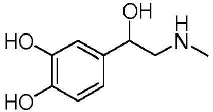 | Epinephrine |
| 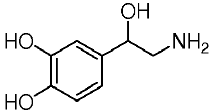 | Norepinephrine |
| 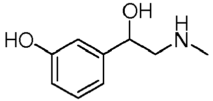 | Phenylephrine |
| 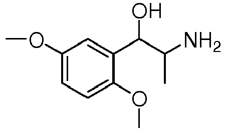 | Methoxamine |

Brimonidine and its pharmaceutically acceptable salts are preferred embodiments of the composition underlying the present invention. Preferably, the active ingredient of the composition for use according to the present invention is brimonidine tartrate.

The alpha-1 adrenergic receptor agonist, alpha-2 adrenergic receptor agonist, or pharmaceutically acceptable salt thereof may be administered alone or in combination with one or more alpha-1 adrenergic receptor agonist, alpha-2 adrenergic receptor agonist, or pharmaceutically acceptable salt thereof. For example, the active ingredients in the pharmaceutical composition may include brimonidine tartrate and oxymetazoline hydrochloride.

Pharmaceutically acceptable salts for each alpha adrenergic receptor agonist are well known in the art. Pharmaceutically acceptable salt means those salts of compounds underlying the composition for use according to the present invention that are safe and effective for topical use in mammals and that possess the desired biological activity. Pharmaceutically acceptable salts include salts of acidic or basic groups present in the compounds underlying the composition for use according to the present invention. Pharmaceutically acceptable acid addition salts include, but are not limited to, hydrochloride, hydrobromide, hydroiodide, nitrate, sulfate, bisulfate, phosphate, acid phosphate, isonicotinate, acetate, lactate, salicylate, citrate, tartrate, pantothenate, bitartrate, ascorbate, succinate, maleate, gentisinate, fumarate, gluconate, glucaronate, saccharate, formate, benzoate, glutamate, methanesulfonate, ethanesulfonate, benzensulfonate, p-toluenesulfonate and pamoate (i.e., 1,1'-methylene-bis-(2-hydroxy-3-naphthoate)) salts. Certain compounds underlying the composition for use according to the present invention can form pharmaceutically acceptable salts with various amino acids. Suitable base salts include, but are not limited to, aluminum, calcium, lithium, magnesium, potassium, sodium, zinc, and diethanolamine salts. Pharmaceutically acceptable salts are discussed in BERGE ET AL., 66 J. PHARM. SCI. 1-19 (1977).

Pharmaceutical compositions include any formulations which are pharmaceutically acceptable for topical delivery of the compounds underlying the composition for use according to the present invention. The choice of topical formulation will depend on several factors, including the physiochemical characteristics of the particular compound(s) and of other excipients present, their stability in the formulation, available manufacturing equipment, and cost constraints.

The pharmaceutically acceptable composition is applied locally to the site of the affected skin of the patient in any conventional manner well known in the art. For example, the composition may be a gel that can be applied to the face and neck of the patient using the fingertips.

The amount of alpha adrenergic receptor agonist applied to the skin is any amount that is effective in treating flushing associated with carcinoid tumors and carcinoid syndrome. Generally the minimum amount of an alpha adrenergic receptor agonist in a topical formulation for use according to the present invention which is to be applied to the affected skin area is about 0.0001 g/cm², preferably about 0.001 g/cm² of skin surface area. The maximum amount of an alpha adrenergic receptor agonist in a topical formulation for use according to the present invention which is to be applied to the affected skin area is about 0.05 g/cm² to about 0.008 g/cm² of skin surface area. Typically, one to four applications per day are recommended during the term of treatment.

Dosages and dosing frequency will be determined by a trained medical professional depending on the activity of the compound underlying the composition for use according to the present invention, the characteristics of the particular topical formulation, and the general physical condition of the person being treated.

In general, each alpha adrenergic receptor agonist or pharmaceutically acceptable salt thereof is present in a formulation for use according to the present invention in a minimum amount of from about 0.05 percent, about 0.1 percent, or about 0.15 percent of the total weight of the formulation. Preferably, an alpha adrenergic receptor agonist or pharmaceutically acceptable salt thereof is present in a formulation for use according to the present invention in a maximum amount of about 5 percent, about 2 percent, about 1 percent, or about 0.5 percent of the total weight of the formulation.

It is to be understood that the present invention contemplates embodiments in which each minima is combined with each maxima to create all feasible ranges. For example, each alpha adrenergic receptor agonist or pharmaceutically acceptable salt thereof may be present in a composition for use according to the present invention in an amount of from about 0.05 percent to about 1 percent based upon the total weight of the composition or, likewise, from about 0.1 percent to about 1 percent based upon the total weight of the composition.

The compounds of the composition for use according to the present invention are to be delivered to the affected area of the skin in a pharmaceutically acceptable carrier. As used herein, a pharmaceutically acceptable carrier is any pharmaceutically acceptable formulation that can be applied to the skin surface for topical or dermal delivery of a pharmaceutical or medicament. The combination of a pharmaceutically acceptable carrier and a compound underlying the composition for use according to the present invention is termed a pharmaceutical composition for use according to the present invention. Pharmaceutical compositions for use according to the present invention are prepared by mixing a compound of the invention with a topical carrier according to well-known methods in the art, for example, methods provided by standard reference texts such as, REMINGTON: THE SCIENCE AND PRACTICE OF PHARMACY 1577-1591, 1672-1673, 866-885(Alfonso R. Gennaro ed. 19th ed. 1995); Ghosh, T. K.; et al. TRANSDERMAL AND TOPICAL DRUG DELIVERY SYSTEMS (1997). See also the discussion of pharmaceutical compositions containing alpha adrenergic receptor agonists in U.S. Patent No. 7,439,241.

The topical carriers useful for topical delivery of compounds underlying the composition for use according to the present invention can be any carrier known in the art for topically administering pharmaceuticals, for example, but not limited to, pharmaceutically acceptable solvents, such as a polyalcohol or water; emulsions (either oil-in-water or water-in-oil emulsions), such as creams or lotions; micro emulsions; gels; ointments; liposomes; powders; and aqueous solutions or suspensions. The preferred carriers are gels, creams, ointments, lotions, and emulsions.

### EXAMPLES

### Example 1

### Gel Composition

| Ingredient | Weight Percent |
|---|---|
| Brimonidine tartrate | 0.33% |
| Carbomer 934P | 1.25% |
| Methylparaben | 0.3% |
| Phenoxyethanol | 0.4% |
| Glycerin | 5.5% |
| 10% Titanium dioxide | 0.625% |
| Propylene glycol | 5.5% |
| 10% NaOH Solution | 6.5% |
| DI Water | QS |
| **TOTAL** | **100%** |

### Example 2

### Cream Composition

| Ingredient | Weight Percent |
|---|---|
| Brimonidine tartrate | 0.5% |
| Oxymetazoline hydrochloride | 0.5% |
| Phenoxyethanol | 0.8% |
| Methylparaben | 0.2% |
| Propylparaben | 0.05% |
| Disodium EDTA | 0.01% |
| Butylated Hydroxytoluene | 0.05% |
| PEG-300 | 4.0% |
| PEG-6 Stearate (and) Glycol Stearate (and) PEG-32 Stearate | 7.5% |
| Cetostearyl alcohol | 4.0% |
| Caprylic capric triglycerides | 7.0% |
| Diisopropyl adipate | 7.0% |
| Oleyl alcohol | 7.0% |
| Lanolin USP | 2.0% |
| Ceteareth-6 (and) Stearyl Alcohol | 2.0% |
| Ceteareth - 25 | 2.0% |
| Tartaric Acid | 0.001% |
| DI Water | 55.389% |
| **TOTAL** | **100%** |

### Example 3

### Lotion Composition

| Ingredient | Weight Percent |
|---|---|
| Brimonidine tartrate | 0.25% |
| Amerlate P Isopropyl Lanolate | 0.5% |
| Stearic Acid | 3.0% |
| Glyceryl Stearate | 2.0% |
| Methyl Gluceth-20 | 5.0% |
| Triethanolamine | 1.0% |
| Water | 83.45% |
| Polyquaternium-24 and Hyaluronic Acid (BIOCARE Polymer HA-24) | 3.8% |
| Germaben IIE | 1.0% |
| **TOTAL** | **100%** |

### Example 4

A gel containing 0.33 weight% brimonidine tartrate is administered to the face and neck of a patient with carcinoid syndrome. The patient applies the gel to his skin twice a day as necessary.

## Claims

1. A pharmaceutical composition for use in the treatment of flushing associated with carcinoid tumors and carcinoid syndrome in a patient in need thereof, which composition comprises an alpha adrenergic receptor agonist, pharmaceutically acceptable salt thereof, or combinations thereof; and a pharmaceutically acceptable carrier, wherein said composition is to be topically applied to the site of the flushing.

2. A composition for use according to claim 1, where the site of the flushing is the face or the neck.

3. A composition for use according to claim 1 or claim 2, wherein the alpha adrenergic receptor agonist is an alpha-1 adrenergic receptor agonist or an alpha-2 adrenergic receptor agonist.

4. A composition for use according to claim 3, wherein the alpha-1 adrenergic receptor agonist or alpha-2 adrenergic receptor agonist is brimonidine, tetrahydrozoline, naphazoline, xylometazoline, epinephrine, norepinephrine, oxymetazoline, phenylephrine, or methoxamine.

5. A composition for use according to claim 3, wherein the alpha-2 adrenergic receptor agonist is brimonidine or pharmaceutically acceptable salts thereof.

6. A composition for use according to any one of the preceding claims, wherein the pharmaceutically acceptable carrier is a gel, cream, ointment, lotion, or emulsion.

7. A composition for use according to any one of the preceding claims, wherein the alpha adrenergic receptor agonist is brimonidine or a pharmaceutically acceptable salt thereof and is present in an amount of from about 0.5% by weight to about 5% by weight of the composition.

8. A composition for use according to any one of the preceding claims, wherein the alpha adrenergic receptor agonist is brimonidine or a pharmaceutically acceptable salt thereof and is present in an amount of from about 0.5% by weight to about 2% by weight of the composition.

9. A composition for use according to claim 1, wherein the carrier is a gel and the alpha adrenergic receptor agonist is brimonidine or a pharmaceutically acceptable salt thereof and is present in an amount of about 0.33% by weight of the gel.

## Patentansprüche

1. Pharmazeutische Zusammensetzung zur Verwendung bei der Behandlung von Erröten, assoziiert mit karzinoiden Tumoren und Karzinoidsyndrom in einem Patienten, der dies benötigt, wobei die Zusammensetzung einen alpha-adrenergen Rezeptoragonisten, ein pharmazeutisch verträgliches Salz davon, oder Kombinationen davon; und einen pharmazeutisch verträglichen Träger umfasst, wobei die Zusammensetzung auf der Stelle des Errötens topisch anzuwenden ist.

2. Zusammensetzung zur Verwendung gemäß Anspruch 1, wobei die Stelle des Errötens das Gesicht oder der Hals ist.

3. Zusammensetzung zur Verwendung gemäß Anspruch 1 oder Anspruch 2, wobei der alpha-adrenerge Rezeptoragonist ein alpha-1-adrenerger Rezeptoragonist oder ein alpha-2-adrenerger Rezeptoragonist ist.

4. Zusammensetzung zur Verwendung gemäß Anspruch 3, wobei der alpha-1-adrenerge Rezeptoragonist oder der alpha-2- adrenerge Rezeptoragonist Brimonidin, Tetrahydrozolin, Naphazolin, Xylometazolin, Epinephrin, Norepinephrin, Oxymetazolin, Phenylephrin oder Methoxamin ist.

5. Zusammensetzung zur Verwendung gemäß Anspruch 3, wobei der alpha-2-adrenerge Rezeptoragonist Brimonidin oder pharmazeutisch verträgliche Salze davon ist.

6. Zusammensetzung zur Verwendung gemäß einem der vorhergehenden Ansprüche, wobei der pharmazeutisch verträgliche Träger ein Gel, Creme, Salbe, Lotion oder Emulsion ist.

7. Zusammensetzung zur Verwendung gemäß einem der vorhergehenden Ansprüche, wobei der alpha-adrenerge Rezeptoragonist Brimonidin oder ein pharmazeutisch verträgliches Salz davon ist und in einer Menge von von etwa 0,5 Gew.-% bis etwa 5 Gew.-% der Zusammensetzung vorhanden ist.

8. Zusammensetzung zur Verwendung gemäß einem der vorhergehenden Ansprüche, wobei der alpha-adrenerge Rezeptoragonist Brimonidin oder ein pharmazeutisch verträgliches Salz davon ist und in einer Menge von von etwa 0,5 Gew.-% bis etwa 2 Gew.-% der Zusammensetzung vorhanden ist.

9. Zusammensetzung zur Verwendung gemäß Anspruch 1, wobei der Träger ein Gel ist und der alpha-adrenerge Rezeptoragonist Brimonidin oder ein pharmazeutisch verträgliches Salz davon ist und in einer Menge von etwa 0,33 Gew.-% des Gels vorhanden ist.

## Revendications

1. Composition pharmaceutique à utiliser dans le traitement des bouffées vasomotrices associées à des tumeurs carcinoïdes et au syndrome carcinoïde chez un patient en ayant besoin, laquelle composition comprenant un agoniste de récepteur alpha-adrénergique, un sel pharmaceutiquement acceptable de celui-ci, ou des combinaisons de ceux-ci ; et un support pharmaceutiquement acceptable, dans lequel ladite composition doit être appliquée localement sur le site des bouffées vasomotrices.

2. Composition à utiliser selon la revendication 1, dans laquelle le site des bouffées vasomotrices est le visage ou le cou.

3. Composition à utiliser selon la revendication 1 ou la revendication 2, dans laquelle l'agoniste de récepteur alpha-adrénergique est un agoniste de récepteur alpha-1 adrénergique ou un agoniste de récepteur alpha-2 adrénergique.

4. Composition à utiliser selon la revendication 3, dans laquelle l'agoniste de récepteur alpha-1 adrénergique ou l'agoniste de récepteur alpha-2 adrénergique est la brimonidine, la tétrahydrozoline, la naphazoline, la xylométazoline, l'épinéphrine, la norépinéphrine, l'oxymétazoline, la phényléphrine ou la méthoxamine.

5. Composition à utiliser selon la revendication 3, dans laquelle l'agoniste de récepteur alpha-2-adrénergique est la brimonidine ou des sels pharmaceutiquement acceptables de celle-ci.

6. Composition à utiliser selon l'une quelconque des revendications précédentes, dans laquelle le support pharmaceutiquement acceptable est un gel, une crème, une pommade, une lotion ou une émulsion.

7. Composition à utiliser selon l'une quelconque des revendications précédentes, dans laquelle l'agoniste de récepteur alpha-adrénergique est la brimonidine ou un sel pharmaceutiquement acceptable de celle-ci et est présent en une quantité d'environ 0,5 % en poids à environ 5 % en poids de la composition.

8. Composition à utiliser selon l'une quelconque des revendications précédentes, dans laquelle l'agoniste de récepteur alpha-adrénergique est la brimonidine ou un sel pharmaceutiquement acceptable de celle-ci et est présent en une quantité d'environ 0,5 % en poids à environ 2 % en poids de la composition.

9. Composition à utiliser selon la revendication 1, dans laquelle le support est un gel et l'agoniste de récepteur alpha-adrénergique est la brimonidine ou un sel pharmaceutiquement acceptable de celle-ci et est présent en une quantité d'environ 0,33 % en poids du gel.
